(19) Europäisches Patentamt
European Patent Office
Office européen des brevets

(11) Numéro de publication: **0 184 471 B1**

(12) **FASCICULE DE BREVET EUROPEEN**

(45) Date de publication du fascicule du brevet: **14.11.90**

(21) Numéro de dépôt: **85400330.8**

(22) Date de dépôt: **22.02.85**

(51) Int. Cl.⁵: **A 61 K 31/565**, A 61 K 31/57, A 61 K 31/58, A 61 K 37/34, A 61 K 45/06 // (A61K31/565, 31:57, 31:58, 31:48, 31:557),(A61K37/34, 31:565, 31:57, 31:58)

(54) **Produit comprenant une substance antiprogestomimétique et une substance utérotonique.**

(30) Priorité: **29.11.84 FR 8418188**

(43) Date de publication de la demande:
**11.06.86 Bulletin 86/24**

(45) Mention de la délivrance du brevet:
**14.11.90 Bulletin 90/46**

(84) Etats contractants désignés:
**AT BE CH DE FR GB IT LI LU NL SE**

(56) Documents cités:
**EP-A-0 057 115**
**EP-A-0 097 572**
**EP-A-0 104 387**
**EP-A-0 116 974**
**EP-A-0 139 608**
**WO-A-83/03099**
**DE-A-2 528 419**

(73) Titulaire: **ROUSSEL-UCLAF**
**35, boulevard des Invalides**
**F-75007 Paris (FR)**

(72) Inventeur: **Bygdeman, Marc**
**Djursholmsvägen 52**
**S-183 51 Täby (SE)**

(74) Mandataire: **Fritel, Hubert et al**
**Département des Brevets ROUSSEL UCLAF B.P. no 9**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

L'invention a pour objet un produit comprenant au moins une substance possédant des propriétés antiprogestérone ou antiprogestomimétiques et au moins une substance différente d'une prostaglandine et possèdant des propriétés utérotoniques.

Dans la recherche tendant à développer des méthodes non chirurgicales d'interruption de l'état de grossesse, diverses expériences ont abouti à des résultats intéressants.

Les prostaglandines ont notamment été expérimentées. C'est ainsi que l'administration, par diverses voies des prostaglandines naturelles ou synthétiques et notamment de certains analogues de la PGE2 et de 1a $PGF_2$ α s'est révélée efficace.

Cependant des effets secondaires, notamment gastro-instestinaux et pelviens, constatés lors de l'utilisation des prostaglandines ont conduit à rechercher d'autres méthodes non chirurgicales d'interruption de l'état de grossesse.

L'utilisation de produits dotés de propriétés antiprogestomimétiques constitue alors une alternative très prometteuse, non chirurgicale, au traitement par les prostaglandines.

Les premiers essais rapportés avec l'utilisation du RU 486 /11β(4-diméthylaminophényl) 17β-hydroxy 17α-(prop-1-ynyl) estra 4,9-dièn 3-one/, EP.0.057.115, ont confirmé le potentiel de la méthode. Cependant, les doses et les méthodes d'administration testées jusqu'à présent n'ont pas permis d'atteindre un pourcentage d'efficacité de 100%.

Dans la demande de brevet européen EP 0 139 608 publiée après le dépôt de la présente demande européenne sont décrites des compositions comportant une association entre un prostaglandine et un anti-progestatif pour déclancher une accouchement ou pour interrompre une grossesse.

Une étude a été effectuée dans laquelle l'action de la substance antiprogestomimétique a été complétée par l'adjonction d'une substance utérotonique.

La présente invention a donc plus particulièrement pour objet un produit comprenant au moins une substance possédant des propriétés differente d'une prostaglandine et possedant des proprietés utérotoniques en tant que produit de combinaison pour une utilisation simultanée, séparée ou étalée dans le temps dans l'interruption de l'état de grossesse.

Les substances possédant la propriété antiprogestomimétique sont très préférentiellement des composés ayant la structure stéroïde et en particulier les stéroïdes substitués en 11 et en 17.

Parmi ces substances, une première classe de composés préférés est constituée par ceux répondant à la formule $I_A$:

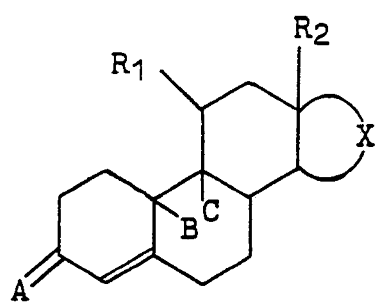

$(I_A)$

dans laquelle $R_1$ représente un radical organique renfermant de 1 à 18 atomes de carbone, contenant au moins un atome d'azote, de phosphore ou de silicium, l'atome immédiatement adjacent au carbone en 11 étant un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation, le groupement C=A en position 3 représente un groupement oxo, libre ou bloqué sous forme de cétal, un groupement

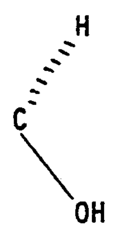

,

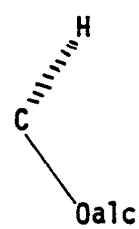

,

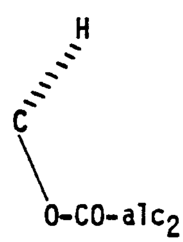

,

un groupement C=NOH, un groupement $C=NO-alc_3$, ou un groupement $CH_2$, $alc_1$, $alc_2$ et $alc_3$ représentant un radical alkyle renfermant de 1 à 8 atomes de carbone ou un groupement aralkyle renfermant de 7 à 15 atomes de carbone et B et C forment ensemble une double liaison ou un pont époxyde, ainsi que leurs sels d'addition avec les acides.

Parmi les composés de formule $I_A$, on préfère ceux dans lesquels le reste X est choisi parmi les cycles de formule:

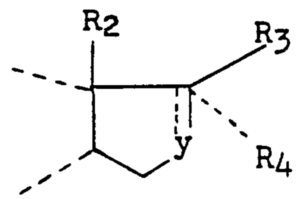

dans laquel $R_2$ conserve la même signification que ci-dessus, le trait pointillé en 16—17 symbolise la présence éventuelle d'une double liaison, Y représente un radical:

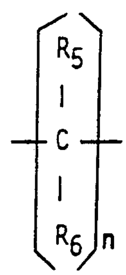

dans lequel n représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, $R_6$, identique ou différent de $R_5$, peut prendre l'une des valeurs indiquées pour $R_5$ et peut également représenter un radical hydroxyle, $R_3$ et $R_4$ identiques ou différents, représentent soit un atome d'hydrogène, soit un radical OH, $Oalc_4$, O—CO—$alc_5$, $alc_4$ et $alc_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

$$-\overset{\overset{\displaystyle O}{\|}}{C}-CH_2OH,$$

soit un radical $-COCH_2OCOalc_6$, dans lequel $alc_6$ représente un radical alkyle renfermant de 1 à 8 atome de carbone éventuellement substitué ou un radical aralkyle renfermant de 7 à 15 atomes de carbone, soit un radical $CO-CO_2H$, ou $CO-CO_2-alc_7$ dans lequel $alc_7$ représente un radical alkyle renfermant de 1 à 8 atomes de carbone, soit un radical:

$$-\overset{\overset{\displaystyle H}{|}}{C}=O,$$

soit un radical

$$-\overset{\overset{\displaystyle NHalc_8}{|}}{C}=O,$$

dans lequel $alc_8$ représente un radical alkyle renfermant de 1 à 8 atomes de carbone ou un radical aralkyle renfermant de 7 à 15 atomes de carbone, soit un radical $-C\equiv N$, soit $R_3$ et $R_4$ forment ensemble

un radical 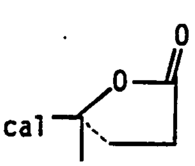 , un radical 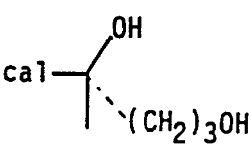

ou un radical 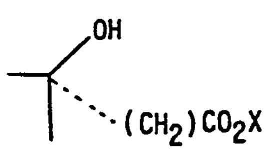

dans lequel X représente un atome d'hydrogène, de sodium ou de potassium.

Une classe plus réduite est constituée par les composés de formule $I_A$ dans laquelle le reste X est choisi parmi les cycles de formule·

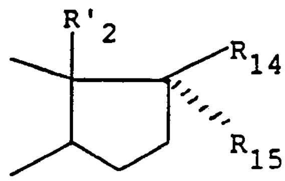

dans laquelle $R'_2$ représente un radical méthyle ou éthyle, $R_{14}$ représente un radical hydroxyle, $R_{15}$ représente un radical alkynyle ayant de 2 à 4 atomes de carbone ou $R_{14}$ et $R_{15}$ forment ensemble un groupement:

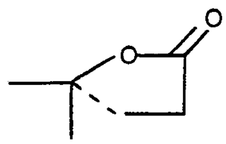 ou 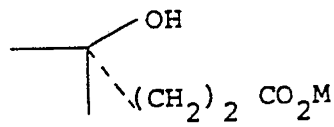

dans lequel M represénte un atome de potassium ou de sodium, et $R_1$ représente un radical 2,3 ou 4-pyridyle,

un radical 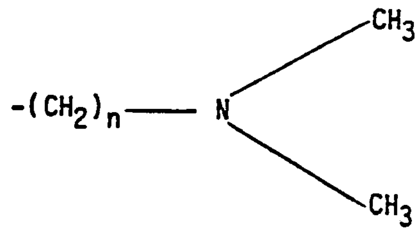 (n≥ 3),

un radical :

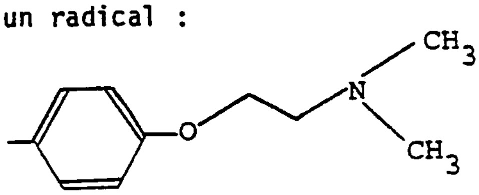 , un radical 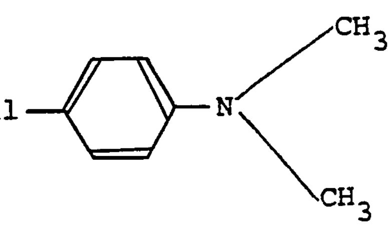

un radical 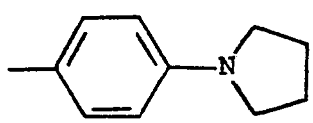 ou un radical 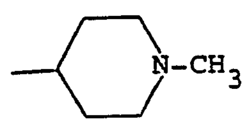;

les atomes d'azote étant éventuellement oxydés.

Ceux-ci ont été décrits principalement dans le brevet européen EP 0.057.115 et pour les composés comportant en 17 une γ lactone ouverte ou fermée ou un radical:

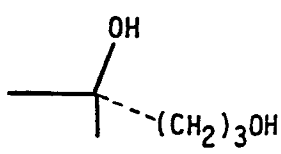

également dans le brevet européen EP 0.116.974.

On trouvera dans ces brevets et notamment dans le brevet 0.057.115 une liste de composés particulièrement préférés et des modes de préparation.

Une deuxième classe de substances antiprogestomimétiques préférées est constituée par les composés de formule $I_B$:

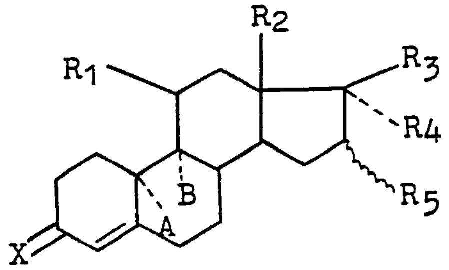

$I_B$

dans laquelle soit $R_1$ représente un radical thiényle éventuellement substitué, un radical furyle; un radical cycloalkyle ayant de 3 à 6 atomes de carbone; un radical phényle éventuellement substitué par un ou plusieurs radicaux choisis parmi les radicaux hydroxy, halogène, trifluorométhyle, alkyle, alkoxy, alkoxyalkyle, alkylthio ou phénylthio éventuellement oxydé sous forme de sulfoxyde ou de sulfone ou alkényloxy ayant au plus 6 atomes de carbone et phényloxy,

soit $R_1$ représente un radical naphtyle ou diphényle ou un radical alkyle ou alkényle portant éventuellement plusieurs insaturations ayant au plus 6 atomes de carbone;

$R_2$ représente un radical méthyle ou éthyle,

$R_3$ représente un atome d'hydrogène, un radical alkyle, alkényle éventuellement substitué, un radical hydroxyle, acétyle, hydroxyacétyle, carboxyalkoxy ayant de 2 à 4 atomes de carbone éventuellement estérifié ou salifié, hydroxyalkyle éventuellement estérifié,

$R_4$ représente soit un atome d'hydrogène, un radical hydroxyle, un radical alkyle, alkényle ou alkynyle au plus 12 atomes de carbone éventuellement substitué par un radical alkylamino, dialkyle amino, ou par un halogène, un radical alkylthio, alkoxy ou trialkylsilyl, soit $R_4$ représente un radical cyanométhyle, soit $R_3$ et $R_4$ représentent

ensemble un radical 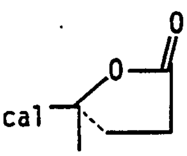 , un radical 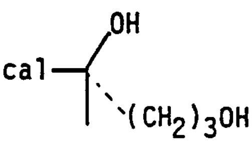

ou un radical 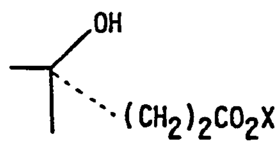

dans lequel X représente un atome d'hydrogène, de sodium ou de potassium.

$R_5$ représente un atome d'hydrogène ou un radical méthyle en position α ou β,

X représente un atome d'oxygène ou un radical hydroxyimino ou alkoxyimino ayant de 1 à 4 atomes de carbone en position syn ou anti;

A et B représentent une fonction α-époxy ou la présence d'une seconde liaison entre les carbones 9 et 10, ainsi que les sels de ces produits avec les acides lorsque $R_4$ représente un radical comportant une fonction amino, et les composés de formule $I'_B$:

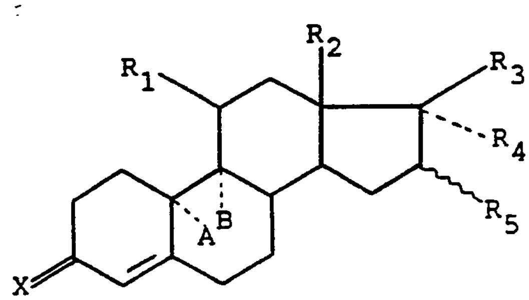

$I'_B$

dans laquelle soit $R_1$ représente un radical thiényle éventuellement substitué, un radical furyle; un radical

cycloalkyle ayant de 3 à 6 atomes de carbone; un radical phényle éventuellement substitué par un ou plusieurs radicaux choisis parmi les radicaux hydroxy, halogene, trifluorométhyle, alkyle, alkoxy, alkylthio éventuellement oxydé sous forme de sulfoxyde ou de sulfone ou alkényloxy ayant au plus 6 atomes de carbone et phényloxy,

soit $R_1$ représente un radical naphtyle ou diphényle ou un radical alkyle ou alkényle portant éventuellement plusieurs insaturations ayant au plus 6 atomes de carbone;

$R_2$ représente un radical méthyle ou éthyle,

$R_3$ représente un atome d'hydrogène un radical alkyle, alkényle éventuellement substitué, un radical hydroxyle, acétyle, hydroxyacétyle, carboxyalkoxy ayant de 2 à 4 atomes de carbone éventuellement estérifié ou salifié, hydroxyalkyle éventuellement estérifié,

$R_4$ représente soit un atome d'hydrogène, un radical hydroxyle, un radical alkyle, alkényle ou alkynyle ayant au plus 12 atomes de carbone éventuellement substitué par un radical alkylamino, dialkyl amino, ou par un halogène, un radical alkylthio, alkoxy ou trialkylsilyl, soit $R_4$ représente un radical cyanométhyle,

$R_5$ représente un atome d'hydrogène ou un radical méthyle en position α ou β,

X représente un atome d'oxygène ou un radical hydroxyimino ou alkoxyimino ayant de 1 à 4 atomes de carbone en position syn ou anti;

A et B représentent une fonction α-époxy ou la présence d'une seconde liaison entre les carbones 9 et 10, ainsi que les sels de ces produits avec les acides lorsque $R_4$ représente un radical comportant une fonction amino.

Parmi les composés de formule $I_B$, on préfère particulièrement ceux de formule $I_B$, dans laquelle le substituent $R_1$ représente un radical phényl substitué en position para par un atome d'halogène, un radical alkyle ayant de 1 à 4 atomes de carbone, un radical alkyloxy, alkylthio ou alkényloxy ayant au plus 4 atomes de carbone, un radical phényle ou phénylthio ou un radical hydroxyle éventuellement acylé, $R_3$ représente un radical hydroxyle et $R_4$ représente un radical alkynyle ayant au plus 4 atomes de carbone, soit $R_3$ et $R_4$

représentent un radical 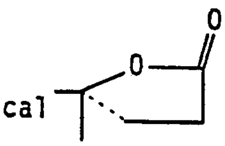 ou 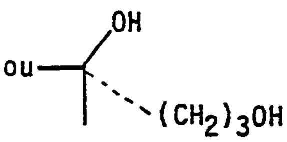 ou 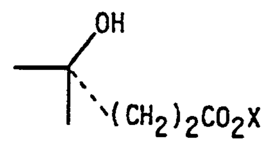

X yant la valeur indiquée ci-dessus.

Les composés de formule $I_B$ ainsi que leurs modes de préparation sont décrits dans les demandes françaises BF 2.522.328 et allemande DE 33 07.143 ainsi que dans les demandes européennes EP. 0.116.974 et 0.104.387.

Parmi les substances de type stéroïde possédant une activité antiprogestomimétique, on peut également citer les composés de formule $I_C$:

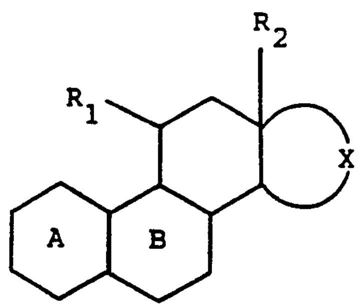

$(I_C)$

dans laquelle $R_1$ représente un radical organique renfermant de 1 à 18 atomes de carbone et éventuellement un ou plusieurs hétéroatomes, l'atome immédiatement adjacent au carbone en 11 étant une atome de carbone, $R_2$ représente un radical hydrocarboné renfermant de 1 à 8 atomes de carbone, X représente le reste d'un cycle pentagonal ou hexagonal éventuellement substitué et éventuellement porteur d'une insaturation, les cycles A et B ayant l'une des structures suivantes:

a) Soit A et B représentent le groupement:

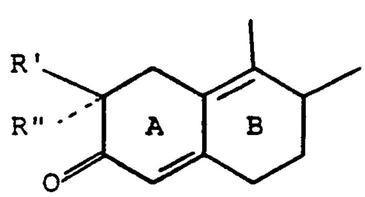

dans lequel R' et R'' identiques ou différents représentent un atome d'hydrogène, un radical nitrile ou un radical alkyle ayant de 1 à 4 atomes de carbone étant étendu que l'un au moins des radicaux R' ou R'' ne représente pas un atome d'hydrogène;

b) Soit A et B représentent le groupement:

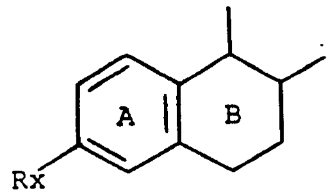

Rx représentent un atome d'hydrogène ou un groupement — ORe dans lequel Re représente un atome d'hydrogène, un radical alkyle ayant de 1 à 6 atomes de carbone éventuellement substitué ou un radical acyle;

c) Soit A et B représentent le groupement:

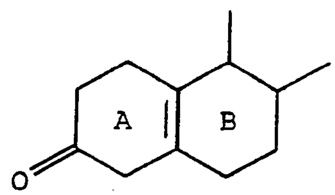

d) Soit A et B représentent le groupement:

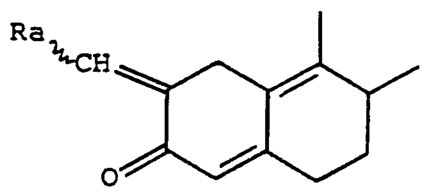

dans lequel Ra représente un radical

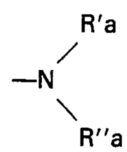

dans lequel R'a et R''a représentent soit un radical alkyle ayant de 1 à 4 atomes de carbone, soit R'a et R''a représentent avec l'atome d'azote auquel ils sont liés, un hétérocycle à 5 ou 6 chaînons comportant éventuellement un autre hétéro atome, ou Ra représente un radical acyloxy, le trait ondulé significant que Ra peut se trouver dans la position E ou Z,

e) Soit A et B représentent le groupement:

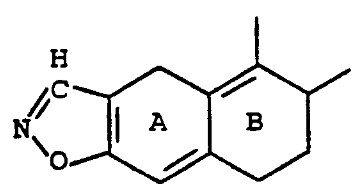

étant entendu que lorsque A et B représentent le groupement:

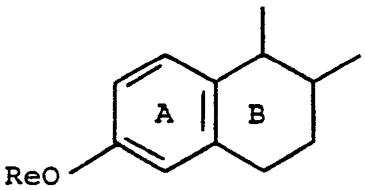

le radical $R_1$ contient au moins un atome d'azote, de phosphore ou de silicium, et que lorsque A et B représentent le groupement:

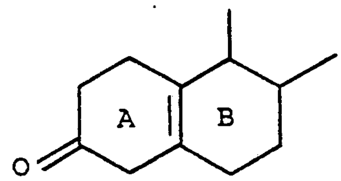

le radical $R_1$ ne représente pas un radical alkyle saturé linéaire, ainsi que les sels d'addition des produits de formule I avec les acides.

Les composés de formule $I_C$ peuvent être préparés selon le procédé indiqué dans la demande européenne 0.097.572.

Parmi les composés particuliers préférés, on peut citer ceux décrits en example dans le brevet européen EP.0.057.115 et la demande allemande DE 33.07.143 et notamment les stéroïdes dont les noms suivent:

11β-/4-(N,N-diméthyl amino éthyloxy) phényl/17β-hydroxy 17α-(prop-1-ynyl) estra 4,9-dièn-3-one;
11β-(4-diméthyl amino phényl) 17β-hydroxy 17α-(prop-1-ynyl) estra 4,9-dièn-3-one;
N-oxyde du 21-chloro 17β-hydroxy 11β-(4-diméthylaminophényl) (17α) 19-nor pregna 4,9-dièn-20-yn-3-one;
N-oxyde du 21-chloro 9α,10α-époxy 17β-hydroxy 11β-(4-diméthylaminophényl) (17α) 19-nor pregn-4-èn 20-yn-3-one;
17β-hydroxy 11β-(4-diméthylaminophényl) 17α-(prop-2-ynyl) estra 4,9-dièn-3-one;
N-oxyde de 17β-hydroxy 11β-(4-diméthylaminophényl) 17α-(prop-1-ynyl) estra 4,9-dièn-3-one.
11β-/(4-chloro) phényl/17β-hydroxy 17α-(prop-1-ynyl) estra 4,9-dièn-3-one;
11β-/(5-chloro) thiényl/17β-hydroxy 17α-(prop-1-ynyl) estra 4,9-dièn-3-one;
11β-/(3-chloro)phényl/17β-hydroxy 17α-(prop-1-ynyl) estra 4,9-dièn-3-one;
11β-/(4-méthylthio) phényl/17β- hydroxy 17α-(prop-1-ynyl) estra 4,9-dièn-3-one;
11β-/(3-fluoro) phényl/17β-hydroxy 17α-(prop-1-ynyl) estra 4,9-dièn-3-one;
11β-/(4-méthylthio) phényl/17α-méthyl 19-nor, pregna 4,9-dièn 3,20-dione;
11β-/(4-méthylthio) phényl/16α-méthyl 19-nor, pregna 4,9-dièn, 3,20-dione;
11β-cyclopropyl 17β-hydroxy 17α-(prop-1-ynyl) estra 4,9-dièn-3-one;
11β-3(2-propényloxy) phényl/17β-hydroxy 17α-(prop-1-ynyl) estra 4,9-dièn 3-one;
9α,10α-époxy 17β-hydroxy 11β-(4-méhoxyphényl) 17α-(prop-1-ynyl)e estr 4-én 3-one;
9α,10α-époxy 17β-hydroxy 11β-/(4-méthylsulfonyl)phényl/17α-(prop-1-ynyl)estra-4-én-3-one;
11β-/(3-fluoro) phényl/3-hydroxyimino 17α-(prop-1-ynyl) estra 4,9-dièn 17β-ol isomère anti;
17β-hydroxy 17α-(prop-1-ynyl) 11β-(4-hydroxyphényl) estra 4,9-dièn 3-one;

Parmi les composés décrits en exemples dans les brevets européens EP.0.116.974 et 0.104.387 qui peuvent être utilisés on peut citer les stéroïdes suivants:

Lactone de l'acide 3-/11β-(4-diméthylaminophényl)-17β-hydroxy-3-oxo-4,9(10)-estradien-17α-yl/-propionique et sel de potassium de l'acide propionique correspondant.
11β-(4-diméthylaminophényl)-17β-hydroxy-17α-(3-hydroxypropyl)4,9(10)-estradien-3-one.
Lactone de l'acide 3-/11β/4-(2-diméthylaminoéthoxy)-phényl/17β-hydroxy 18-méthyl-3-oxo-4,9(10-estradien-17α-yl/-propionique et sel de potassium de l'acide propionique correspondant.
11β-/4-(2-diméthylaminoéthoxy)-phényl)/17β-hydroxy-17α-(3-hydroxypropyl)-18-méthyl-4,9(10)-estradien-3-one.
17α-éthynyl-11β-(4-alloxyphényl)17β-hydroxy-4,9-estradien-3-one.
11β-(4-alloxyphényl)-17β-hydroxy-17α-(1-propynyl)-4,9-estradien-3-one.

L'invention a cependant pour objet préféré l'utilisation comme substance antiprogestomimétique du RU486 de formule indiquée ci-dessus.

Dans le cadre de la présente invention, on peut utiliser comme substance utérotonique, l'une au moins des substances comprises parimi l'ocytocine, les alcoloïdes de l'ergot de seigler, la spartéine.

Parmi les alcaloïdes de l'ergot de seigle, on peut citer plus particulièrement la méthylergométrine, éventuellement sous forme de maléate, la spartéine peut être sous forme de sulfate.

Dans un mode préféré d'administration, le ou les substances possédant des propriétées antiprogestomimétiques et le ou les substances possèdant des propriétés utérotoniques sont administrés de façon séparée.

La substance possédant des propriétés antiprogestomimétiques peut de préférence être administrée par voie orale, mais on peut également l'administrer par voie intra-musculaire sous-cutanée ou transmuqueuse, notamment vaginale ou rectale.

La substance possédant des propriétés utérotnioques peut de préférence être administrée par voie intra-musculaire, mais on peut également l'administrer par voie intra-veineuse, sous-cutanée, orale ou vaginale.

Une méthode d'administration de la substance possédant des propriétés antiprogestomimétiques consiste à l'administrer pendant plusieurs jours consécutifs, de préférence de 1 à 8 jours et de préférence encore de 1 à 4 jours.

La substance possédant les propriétés utérotoniques est administrée de façon moins prolongée et un mode d'administration préféré consiste en une ou plusieurs administrations sur une courte durée, administrations qui peuvent être faites pendant le traitement par la substance antiprogestomimétique, mais de préférence à la fin de ce traitement.

L'invention a donc particulièrement pour objet un produit tel que décrit ci-dessus caractérisé en ce que la substance possédant des propriétés antiprogestomimétiques est administrée par voie orale pendant 1 à 4 jours et en ce que la substance possédant des propriétés utérotoniques est administrée sous forme d'une injection unique se situant à la fin du traitement par la substance antiprogestomimétique.

La substance antiprogestomimétique est administrée de préférence sous forme de comprimés, mais elle peut être administrée sous les formes galéniques usuelles.

On trouvera une énumération de ces différentes présentations, notamment dans la demande de brevet européen 0.57.115 à la page 8, linge 31 et suivantes.

Les comprimés de substance antiprogestomimétique peuvent, par exemple, être dosés à 25 ou 50 mg.

La posologie journalière peut varier entre 10 et 800 mg, de préférence entre 25 et 200 mg par voie orale.

La substance utérotonique est administrée de préférence sous forme d'injection intra-musculaire à la fin de l'administration de la substance antiprogestomimétique.

Les produits de la présente invention trouvent leur application préférentielle dans l'interruption des grossesses précoces, c'est-à-dire de moins de 10 semaines et de préférence encore dans le cas de grossesses de 7 semaines au plus (c'est-à-dire jusqu'à 49 jours après le début des dernières règles). Cependant, les produits de la présente invention peuvent également être utilisés dans le cas d'interruption de grossesses évolués.

On trouvera ci-après les résultats d'une expérimentation pharmacologique effectuée avec les produits de l'invention.

Etude Pharmacologique

L'étude a porté sur 33 jeunes femmes en bonne santé présentant une grossesse apparemment normale et une aménorhée de 49 jours a plus.

Le composé RU 486 a été administré oralement à différentes doses:

1) 25 mg 2 fois par jour pendant 4 jours.

2) 25 mg 4 fois par jour pendant 4 jours.

Chez 16 patientes une injection intramusculaire de 0,25 mg de la méthylsulfonylamide de la 16-phénoxytétranor PG $E_2$ (sulprostone) a été administrée le matin du dernier jour de traitement par le RU 486. Les sujets ont jeuné au moins heure avant jusqu'à une heure après la prise du produit.

Les résultats obtenus sont rapportés dans le tableau I ci-après.

TABLEAU I

| Groupe de Patientes | Dose(mg) RU 486 | Nombre de patientes | Avortements complets | Avortements incomplets | Poursuite de la grossesse |
|---|---|---|---|---|---|
| Administration du RU 486 seul GROUPE A | 25 x 2 | 11 | 8 | 1 | 2* |
| | 25 x 4 | 6 | 5 | 0 | 1 |
| Total | | 17 | 13 (76,5%) | 1 (5,9%) | 3 (17,6%) |
| Administration du RU 486 + 0,25 mg de sulprostone par injection intramusculaire le dernier jour du traitement GROUPE B | 25 x 2 | 9 | 9 | 0 | 0 |
| | 25 x 4 | 7 | 7 | 0 | 0 |
| Total | | 16 | 16 (100%) | 0 | 0 |

* dont une grosse extra-utêrine.

*Conclusion:* Lors de l'étude pharmacologique rapportée, le produit comprenant le RU 486 et la SULPROSTONE a permis d'obtenir un pourcentage de 100% d'avortements alors que les mêmes quantités de RU 486 administré seul n'ont permis d'obtenir qu'un pourcentage de 76,5%. De plus, aucun saignement excessif n'a été observé dans le groupe de patientes recevant l'association de produits.

Enfin l'administration du RU 486 et de la SULPROSTONE n'a pas donné lieu à des effets secondaires gastrointestinaux comme ceux fréquemment observés avec le traitement par les prostaglandines seules.

(voir par exemple Bygdeman Menstrual regulation with prostaglandins in Practical Application of Prostaglandins and their Synthesis Inhibitors 1979 p. 267—282 (Lancaster MTP Press).

**Revendications**

1. Produit comprenant au moins une substance possédant des propriétés antiprogestérone ou anti-progestomimétiques et au moins une substance différente d'une prostaglandine et possédant des propriétées utérotoniques en tant que produit de combinaison, pour une utilisation simultanée, séparée ou étalée dans le temps dans l'interruption de l'état de grossesse.

2. Produit selon la revendication 1 caractérisé en ce qu'il comprend, comme substance possédant des propriétés anti-progestomimétiques, au moins un composé répondant à la formule $I_A$:

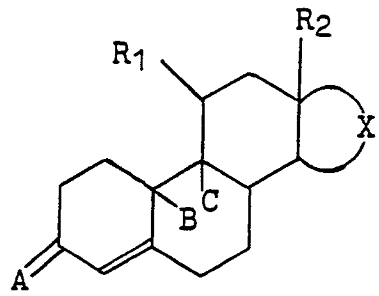

$(I_A)$

dans laquelle $R_1$ représente un radical organique renfermant de 1 à 18 atomes de carbone, contenant au moins un atome d'azote, de phosphore ou de silicium, l'atome immédiatement adjacent au carbone en 11 étant un atome de carbone, $R_2$ représente un radical hydrocarboné renfermant de 1 à 8 atomes de carbone, X représente le reste d'un cycle pentagonal ou hexagonal éventuellement substitué et éventuellement porteur d'insaturation, le groupement C=A en position 3 représente un groupement oxo, libre ou bloqué sous forme de cétal, un groupement

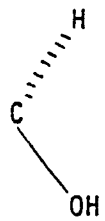 , 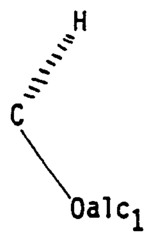 , 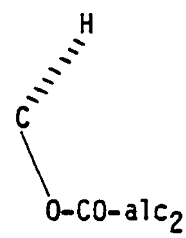 ,

une groupement C=NOH, un groupement C=NO-alc$_3$, ou un groupement $CH_2$, alc$_1$, alc$_2$ et alc$_3$ représentant un radical alkyle renfermant de 1 à 8 atomes de carbone ou un groupement aralkyle renfermant de 7 à 15 atomes de carbone et B et C forment ensemble une double liaison ou un pont époxyde, ainsi que leurs sels d'addition avec les acides.

3. Produit selon les revendications 1 ou 2 caractérisé en ce que, dans la formule $I_A$, le reste X est choisi parmi les cycles de formule:

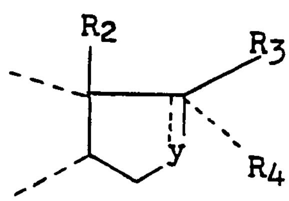

dans laquel $R_2$ conserve la même signification que dans la revendication 2, le trait pointillé en 16—17 symbolise la présence éventuelle d'une double liaison, Y représente un radical:

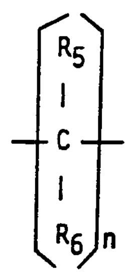

dans lequel n représente le nombre 1 ou 2, $R_5$ représente un atome d'hydrogène, un radical alkyle renfermant de 1 à 8 atomes de carbone, un radical alkényle ou alkynyle renfermant de 2 à 8 atomes de carbone, un radical aryle renfermant de 6 à 14 atomes de carbone ou un radical aralkyle renfermant de 7 à 15 atomes de carbone, $R_6$, identique ou différent de $R_5$, peut prendre l'une des valeurs indiquées pour $R_5$ et peut également représenter un radical hydroxyle, $R_3$ et $R_4$ identiques ou différents, représentent soit un atome d'hyrogène, soit un radical OH, Oalc$_4$, O—CO—alc$_5$, alc$_4$ et alc$_5$ représentant un radical alkyle renfermant de 1 à 8 atomes de carbone ou aralkyle renfermant de 7 à 15 atomes de carbone, soit un radical alkényle ou alkynyle renfermant de 2 à 8 atomes de carbone, soit un radical

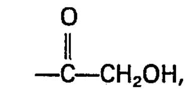

soit un radical $-COCH_2OCOalc_6$, dans lequel $alc_6$ représente un radical alkyle renfermant de 1 à 8 atomes de carbone éventuellement substitué ou un radical aralkyle renfermant de 7 à 15 atomes de carbone, soit un radical $CO-CO_2H$, ou $CO-CO_2-alc_7$ dans lequel $alc_7$ représente un radical alkyle renfermant de 1 à 8 atomes de carbone, soit un radical:

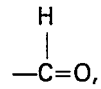

soit un radical

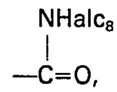

dans lequel $alc_8$ représente un radical alkyle renfermant de 1 à 8 atomes de carbone ou un radical aralkyle renfermant de 7 à 15 atomes de carbone, soit un radical $-C{\equiv}N$, soit $R_3$ et $R_4$ forment ensemble

un radical 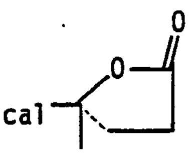, un radical 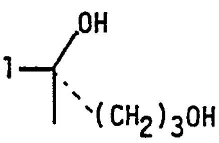

ou un radical 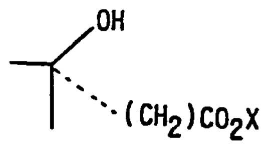

dans lequel X représente un atome d'hydrogène, de sodium ou de potassium.

4. Produit selon les revendications 1 à 3 caractérisé en ce que, dans la formule $I_A$, le reste X est choisi parmi les cycles de formule:

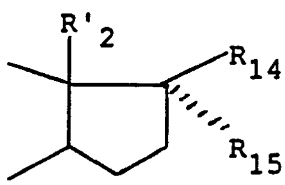

dans laquelle $R'_2$ représente un radical méthyle ou éthyle, $R_{14}$ représente un radical hydroxyle, $R_{15}$ représente un radical alkynyle ayant de 2 à 4 atomes de carbone ou $R_{14}$ et $R_{15}$ forment ensemble un groupement:

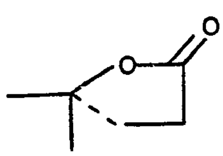

ou

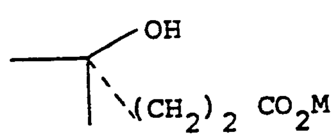

dans lequel M représente un atome de potassium ou de sodium.

5. Produit selon les revendications 1 à 4 caractérisé en ce que, dans la formule $I_A$, $R_1$ représente un radical 2,3 ou 4-pyridyle, un radical

un radical 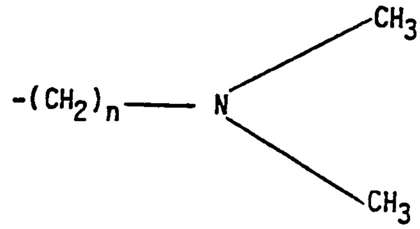 $(n \geqslant 3)$,

un radical :

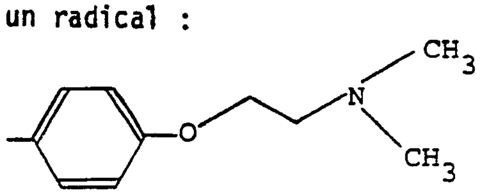 , un radical 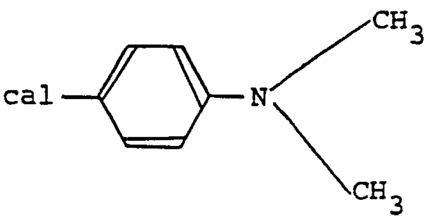

un radical 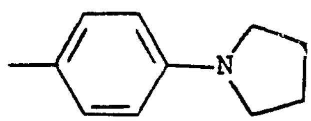 ou un radical 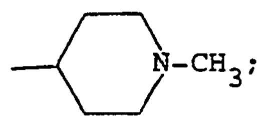;

les atomes d'azote étant éventuellement oxydés.

6. Produit selon la revendication 1, caractérisé en ce qu'il comprend, comme substance possédant des propriétés anti-progestomimétiques, au moins un composé répondant à la formule $I_B$:

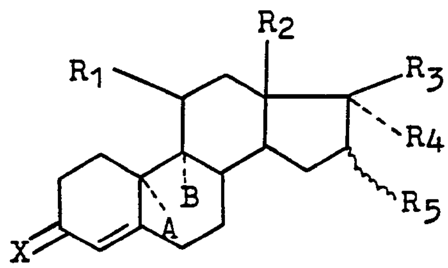

$I_B$

dans laquelle soit $R_1$ représente un radical thiényle éventuellement substitué, un radical furyle; un radical cycloalkyle ayant de 3 à 6 atomes de carbone; un radical phényle éventuellement substitué par un ou plusieurs radicaux choisis parmi les radicaux hydroxy, halogène, trifluorométhyle, alkyle, alkoxy, alkoxy-alkyle, alkylthio ou phénylthio éventuellement oxydé sous forme de sulfoxyde ou de sulfone ou alkényloxy ayant au plus 6 atomes de carbone et phényloxy,

soit $R_1$ représente un radical naphtyle ou diphényle ou un radical alkyle ou alkényle portant éventuellement plusieurs insaturations ayant au plus 6 atomes de carbone;

$R_2$ représente un radical méthyle ou éthyle,

$R_3$ représente un atome d'hydrogène, un radical alkyle, alkényle éventuellement substitué, un radical hydroxyle, acétyle, hydroxyacétyle, carboxyalkoxy ayant de 2 à 4 atomes de carbone éventuellement estérifié ou salifié, hydroxyalkyle éventuellement estérifié,

$R_4$ représente soit un atome d'hydrogène, un radical hydroxyle, un radical alkyle, alkényle ou alkynyle ayant au plus 12 atomes de carbone éventuellement substitué par un radical alkylamino, dialkyle amino, ou par un halogène, un radical alkylthio, alkoxy ou trialkylsilyl, soit $R_4$ représente un radical cyanométhyle, soit $R_3$ et $R_4$ représentent

ensemble un radical 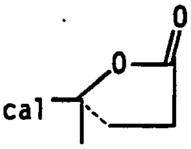 , un radical 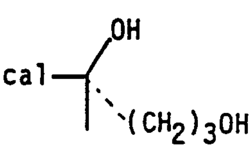

ou un radical 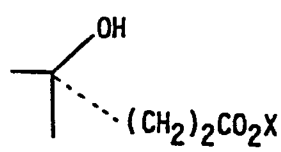

dans lequel X représente un atome d'hydrogène, de sodium ou de potassium.

$R_5$ représente un atome d'hydrogène ou un radical méthyle en position α ou β,

X représente un atome d'oxygène ou un radical hydroxyimino ou alkoxyimino ayant de 1 à 4 atomes de carbone en position syn ou anti;

A et B représentent une fonction α-époxy ou la présence d'une seconde liaison entre les carbones 9 et 10, ainsi que les sels de ces produits avec les acides lorsque $R_4$ représente un radical comportant une fonction amino.

7. Produit selon les revendications 1 et 6 caractérisé en ce qu'il comprend, comme substance possédant des propriétés antiprogestomimétiques, au moins un composé répondant à la formule $I'_B$:

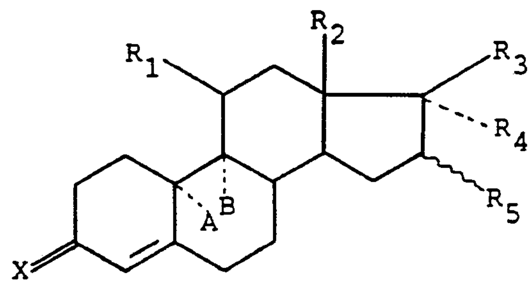

$I'_B$

dans laquelle soit $R_1$ représente un radical thiényle éventuellement substitué, un radical furyle; un radical cycloalkyle ayant de 3 à 6 atomes de carbone; un radical phényle éventuellement substitué par un ou plusieurs radicaux choisis parmi les radicaux hydroxy, halogène, trifluorométhyle, alkyle, alkoxy, alkoxy-alkyle, alkylthio ou phénylthio éventuellement oxydé sous forme de sulfoxyde ou de sulfone ou alkényloxy ayant au plus 6 atomes de carbone et phényloxy,

soit $R_1$ représente un radical naphtyle ou diphényle ou un radical alkyle ou alkényle portant éventuellement plusieurs insaturations ayant au plus 6 atomes de carbone;

$R_2$ représente un radical méthyle ou éthyle,

$R_3$ représente un atome d'hydrogène, un radical alkyle, alkényle éventuellement substitué, un radical hydroxyle, acétyle, hydroxyacétyle, carboxyalkoxy ayant de 2 à 4 atomes de carbone éventuellement estérifié ou salifié, hydroxyalkyle éventuellement estérifié,

$R_4$ représente soit un atome d'hydrogène, un radical hydroxyle, un radical alkyle, alkényle ou alkynyle ayant au plus 12 atomes de carbone éventuellement substitué par un radical alkylamino, dialkyl amino, ou par un halogène, un radical alkylthio, alkoxy ou trialkylsilyl, soit $R_4$ représente un radical cyanométhyle,

$R_5$ représente un atome d'hydrogène ou un radical méthyle en position α ou β,

X représente un atome d'oxygène ou un radical hydroxyimino ou alkoxyimino ayant de 1 à 4 atomes de carbone en position syn ou anti;

A et B représentent une fonction α époxy ou la présence d'une seconde liaison entre les carbones 9 et 10, ainsi que les sels de ces produits avec les acides lorsque $R_4$ représente un radical comportant une fonction amino.

8. Produit selon les revendications 6 et 7 caractérisé en ce que le substituant $R_1$ représente un radical phénylsubstitué en position para par un atome d'halogène, un radical alkyle ayant de 1 à 4 atomes de carbone, un radical alkyloxy, alkylthio ou alkényloxy ayant au plus 4 atomes de carbone, un radical phényle ou phénylthio ou un radical hydroxyle éventuellement acylè,

$R_3$ représente un radical hydroxyle et $R_4$ représente un radical alkynyle ayant au plus 4 atomes de carbone, soit $R_3$ et $R_4$

représentent un radical 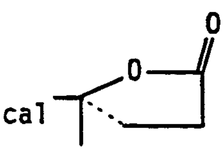 ou 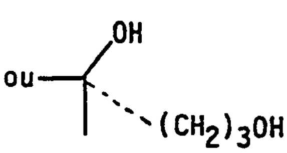 ou 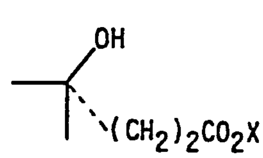

X ayant la valeur indiquée à la revendication 6.

9. Produit selon la revendication 1 caractérisé en ce qu'il comprend, comme substance possedant des propriétés antiprogestomimétiques, au moins un stéroïde choisi parmi les composés suivants:

11β-/4-(N,N-diméthyl amino éthyloxy) phényl/17β-hydroxy 17α-(prop-1-ynyl) estra 4,9-dièn-3-one;

11β-(4-diméthyl amino phényl) 17β-hydroxy 17α-(prop-1-ynyl) estra 4,9-dièn-3-one;

N-oxyde du 21-chloro 17β-hydroxy 11β-(4-diméthylaminophényl) (17α) 19-nor pregna 4,9-dièn-20-yn-3-one;

N-oxyde du 21-chloro 9α,10α-époxy 17β-hydroxy 11β-(4-diméthylaminophényl) (17α) 19-nor pregn-4-èn 20-yn-3-one;

17β-hydroxy 11β-(4-diméthylaminophényl) 17α-(prop-2-ynyl) estra 4,9-dièn-3-one;
N-oxyde de 17β-hydroxy 11β-(4-diméthylaminophényl) 17α-(prop-1-ynyl) estra 4,9-dièn-3-one.
11β-/(4-chloro) phényl/17β-hydroxy 17α-(prop-1-ynyl) estra 4,9-dièn-3-one;
11β-/(5-chloro) thiényl/17β-hydroxy 17α-(prop-1-ynyl) estra 4,9-dièn-3-one;
11β-/(3-chloro)phényl/17β-hydroxy 17α-(prop-1-ynyl) estra 4,9-dièn-3-one;
11β-/(4-méthylthio) phényl/17β- hydroxy 17α-(prop-1-ynyl) estra 4,9-dièn-3-one;
11β-/(3-fluoro) phényl/17β-hydroxy 17α-(prop-1-ynyl) estra 4,9-dièn-3-one;
11β-/(4-méthylthio) phényl/17α-méthyl 19-nor, pregna 4,9-dièn 3,20-dione;
11β-/(4-méthylthio) phényl/16α-méthyl 19-nor, pregna 4,9-dièn, 3,20-dione;
11β-cyclopropyl 17β-hydroxy 17α-(prop-1-ynyl) estra 4,9-dièn-3-one;
11β-3(2-propényloxy) phényl/17β-hydroxy 17α-(prop-1-ynyl) estra 4,9-dièn 3-one;
9α,10α-époxy 17β-hydroxy 11β-(4-méthoxyphényl) 17α-(prop-1-ynyl)e estr 4-én 3-one;
9α,10α-époxy 17β-hydroxy 11β-/(4-méthylsulfonyl)phényl/17α-(prop-1-ynyl)estra-4-én-3-one;
11β-/(3-fluoro) phényl/3-hydroxyimino 17α-(prop-1-ynyl) estra 4,9-dièn 17β-ol isomère anti;
17β-hydroxy 17α-(prop-1-ynyl) 11β-(4-hydroxyphényl) estra 4,9-dièn 3-one;
Lactone de l'acide 3-/11β-(4-diméthylaminophényl)-17β-hydroxy-3-oxo-4,9(10)-estradien-17α-yl/-propionique et sel de potassium de l'acide propionique correspondant.
11β-(4-diméthylaminophényl)-17β-hydroxy-17α-(3-hydroxypropyl)4,9(10)-estradien-3-one.
Lactone de l'acide 3-/11β/4-(2-diméthylaminoéthoxy)-phényl/17β-hydroxy 18-méthyl-3-oxo-4,9(10-estradien-17α-yl/-propionique et sel de potassium de l'acide propionique correspondant.
11β-/4-(2-diméthylaminoéthoxy)-phényl)/17β-hydroxy-17α-(3-hydroxypropyl)-18-méthyl-4,9(10)-estradien-3-one.
17α-éthynyl-11β-(4-alloxyphényl)17β-hydroxy-4,9-estradien-3-one.
11β-(4-alloxyphényl)-17β-hydroxy-17α-(1-propynyl)-4,9-estradien-3-one.

10. Produit selon les revendications 1 et 9 caractérisé en ce qu'il comprend, comme substance possédant des propriétés antiprogestomimétiques, le composé répondant à la formule:
11β-(4-diméthylaminophényl) 17β-hydroxy 17α-(prop-1-ynyl) estra 4,9-diène-3-one.

11. Produit selon la revendication 1 caractérisé en ce qu'il comprend, comme substance possédant des propriétés utérotoniques, l'une au moins des substances comprises parmi l'ocytocine, les alcoloïdes de l'ergot de seigle et la spartéine.

**Patentansprüche**

1. Produkt, enthaltend zumindest eine Substanz mit Anti-Progesteroneigenschaften oder anti-progestomimetischen Eigenschaften und zumindest eine Substanz, die von einen Prostaglandin verschieden ist und uterotonische Eigenschaften besitzt, als Kombinationsprodukt für eine gleichzeitige, getrennte oder zeitlich verschobene Verwendung bei der Unterbrechung der Schwangerschaft.

2. Produkt gemäß Anspruch 1, dadurch gekennzeichnet, daß es als Substanz mit anti-progestomimetischen Eigenschaften zumindest eine Verbindung der Formel $I_A$

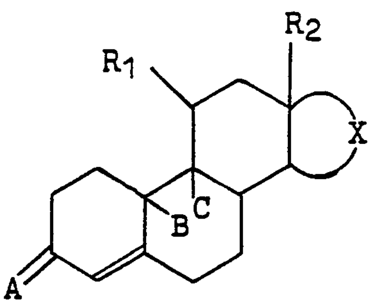

$(I_A)$

enthält, worin $R_1$ einen organischen Rest mit 1 bis 18 Kohlenstoffatomen mit zumindest einen Stickstoff-, Phosphor- oder Siliciumatom bedeutet, wobei das dem Kohlenstoff in 11-Stellung unmittelbar benachbarte Atom ein Kohlenstoffatom ist, $R_2$ eine Kohlenwasserstoffrest mit 1 bis 8 Kohlenstoffatomen wiedergibt, X für den Rest eines gegebenenfalls substituierten und gegebenenfalls eine Unsättigung enthaltenden, pentagonalen oder hexagonalen Rings steht, die Gruppe C=A in 3-Stellung eine freie oder in Form des Ketals blockierte Oxogruppe, eine Gruppe

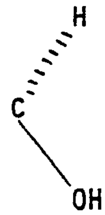 , 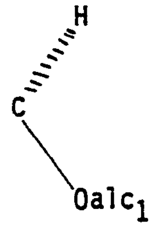 , 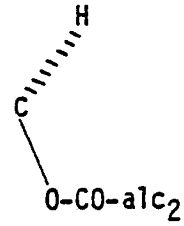 ,

eine Gruppe C=NOH, eine Gruppe C=NO—$alc_3$ oder eine Gruppe $CH_2$ bedeutet, wobei $alc_1$, $alc_2$ und $alc_3$ für eine Akylgruppe mit 1 bis 8 Kohlenstoffatomen oder eine Aralkylgruppe mit 7 bis 15 Kohlenstoffatomen stehen und B und C gemeinsam eine Doppelbindung oder eine Epoxidbrücke bilden, sowie deren Additionssalze mit Säuren.

3. Produkt gemäß den Ansprüchen 1 oder 2, dadurch gekennzeichnet, daß in der Formel $I_A$ der Rest X unter den Ringen der Formel

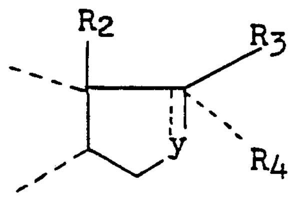

ausgewählt ist, worin $R_2$ die in Anspruch 2 angegebene Bedeutung besitzt, die gepunktete Linie in 16-17-Stellung die etwaige Anwesenheit einer Doppelbindung symbolisiert, Y einen Rest

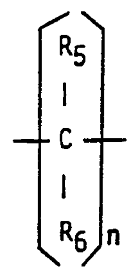

wiedergibt, worin n die Zahl 1 oder 2 bedeutet, $R_5$ für ein Wasserstoffatom, einen Alkylrest mit 1 bis 8 Kohlenstoffatomen, einen Alkenyl- oder Alkinylrest mit 2 bis 8 Kohlenstoffatomen, eine Arylgruppe mit 6 bis 14 Kohlenstoffatomen oder eine Aralkylgruppe mit 7 bis 15 Kohlenstoffatomen steht, $R_6$ identisch mit oder verschieden von $R_5$ eine der für $R_5$ angegebenen Bedeutungen annehmen kann und auch einen Hydroxylrest bedeuten kann, $R_3$ und $R_4$, gleich oder voneinander verschieden, entweder ein Wasserstoffatom oder einen Rest OH, $Oalc_4$, O—CO—$alc_5$, wobei $alc_4$ und $alc_5$ einen Alkylrest mit 1 bis 8 Kohlenstoffatomen oder einen Aralkylrest mit 7 bis 15 Kohlenstoffatomen wiedergeben, oder einen Alkenyl- oder Alkinylrest mit 2 bis 8 Kohlenstoffatomen oder einen Rest

O
‖
—C—$CH_2OH$

oder einen Rest —$COCH_2OCOalc_6$, worin $alc_6$ eine Alkylgruppe mit 1 bis 8 Kohlenstoffatomen, die gegebenenfalls substituiert ist, oder eine Aralkylgruppe mit 7 bis 15 Kohlenstoffatomen wiedergibt, oder einen Rest CO—$CO_2H$ oder CO—$CO_2$-$alc_7$, worin $alc_7$ eine Alkylgruppe mit 1 bis 8 Kohlenstoffatomen bedeutet oder einen Rest

H
|
—C=O,

oder einen Rest

$NHalc_8$
|
—C=O,

worin $alc_8$ einen Alkylrest mit 1 bis 8 Kohlenstoffatomen oder einen Aralkylrest mit 7 bis 15 Kohlenstoffatomen wiedergibt, oder einen Rest —C≡N bedeuten oder $R_3$ und $R_4$ gemeinsam

einen Rest 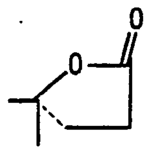, einen Rest 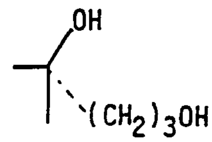

oder einen Rest 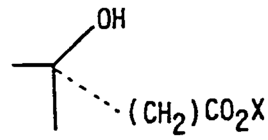

worin X für eine Wasserstoff-, Natrium- oder Kaliumatom steht, bilden.

4. Produkt gemäß den Ansprüchen 1 bis 3, dadurch gekennzeichnet, daß in der Formel $I_A$ der Rest X ausgewählt ist unter den Ringen der Formel

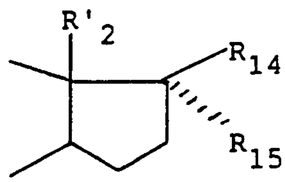

worin $R'_2$ für eine Methyl- oder Ethylgruppe steht, $R_{14}$ eine Hydroxylgruppe bedeutet, $R_{15}$ eine Alkinylgruppe mit 2 bis 4 Kohlenstoffatomen bedeutet oder $R_{14}$ und $R_{15}$ gemeinsam eine Gruppe

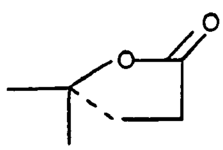 oder 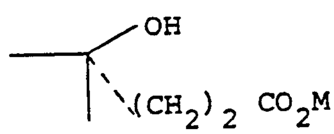

bilden, worin M ein Kalium- oder Natriumatom bedeutet.

5. Produkt gemäß den Ansprüchen 1 bis 4, dadurch gekennzeichnet, daß in der Formel $I_A$ $R_1$ einen 2-, 3- oder 4-Pyridylrest, einen Rest

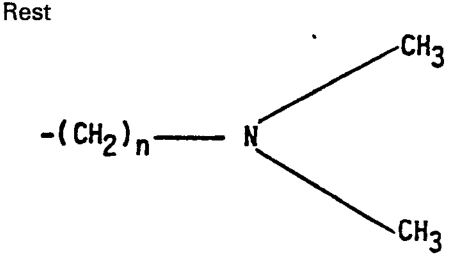 (n ≥ 3),

einen Rest

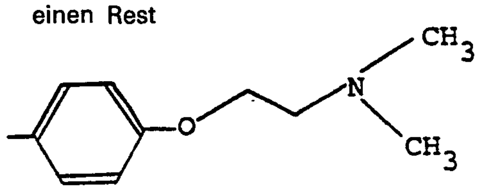, einen Rest 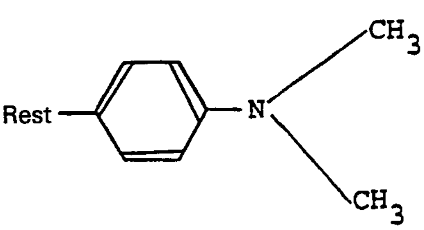

oder Rest 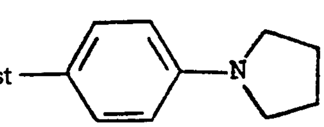 einen Rest 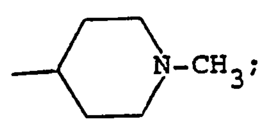;

bedeutet, wobei die Stickstoffatome gegebenenfalls oxidiert sind.

6. Produkt gemäß Anspruch 1, dadurch gekennzeichnet, daß es als Substanz mit anti-progestomimetischen Eigenschaften zumindest eine Verbindung der Formel $I_B$

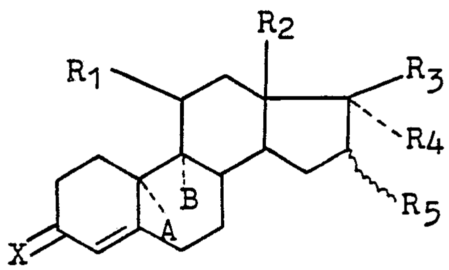

$I_B$

enthält, worin entweder $R_1$ für einen gegebenenfalls substituierten Thienylrest, einen Furylrest, einen Cycloalkylrest mit 3 bis 6 Kohlenstoffatomen, einen Phenylrest, gegebenenfalls substituiert durch einen oder mehrere Reste, ausgewählt unter den Hydroxy-, Halogen-, Trifluormethyl, Alkyl-, Alkoxy-, Alkoxyalkyl-, Alkylthio- oder Phenylthioresten, die gegebenenfalls in Form des Sulfoxids oder Sulfons oxidiert sind, oder Alkenyloxyresten mit höchstens 6 Kohlenstoffatomen und Phenyloxy, steht, oder $R_1$ einen Naphthyl- oder Diphenylrest oder einen Alkyl- oder Alkenylrest, der gegebenenfalls mehrere Unsättigungen enthält, mit höchstens 6 Kohlenstoffatomen bedeutet;

$R_2$ für eine Methyl- oder Ethylgruppe steht;

$R_3$ ein Wasserstoffatom, eine gegebenenfalls substituierte Alkyl- oder Alkenylgruppe, eine Hydroxyl-, Acetyl- oder Hydroxyacetylgruppe, eine Carboxyalkoxygruppe mit 2 bis 4 Kohlenstoffatomen, die gegebenenfalls verestert oder in ein Salz überführt ist, oder gegebenenfalls veresterte Hydroxyalkylgruppe bedeutet;

$R_4$ entweder ein Wasserstoffatom, eine Hydroxylgruppe, eine Alkyl-, Alkenyl- oder Alkinylgruppe mit höchstens 12 Kohlenstoffatomen, die gegebenenfalls durch eine Alkylamino- oder Dialkylaminogruppe oder durch ein Halogenatom, eine Alkylthio-, Alkoxy- oder Trialkylsilylgruppe substituiert ist, bedeutet oder $R_4$ einen Cyanomethylrest wiedergibt oder $R_3$ und $R_4$ gemeinsam

einen Rest 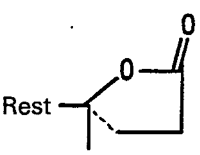, einen Rest 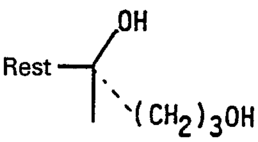

oder einen Rest 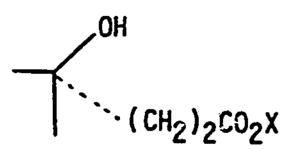

bilden, worin X für ein Wasserstoff-, Natrium- oder Kaliumatom steht, $R_5$ ein Wasserstoffatom oder eine Methylgruppe in α- oder β-Stellung bedeutet, X für ein Sauerstoffatom oder eine Hydroxyimino- oder Alkoxyiminogruppe mit 1 bis 4 Kohlenstoffatomen in syn- oder anti-Stellung steht, A und B eine α-Epoxy-Funktion oder die Anwesenheit einer zweiten Bindung zwischen den Kohlenstoffatomen in 9- und 10-Stellung wiedergeben, sowie die Salze dieser Produkte mit Säuren, wenn $R_4$ einen Rest mit einer Amino-Funktion bedeutet.

7. Produkt gemäß den Ansprüchen 1 und 6, dadurch gekennzeichnet, daß es als Substanz mit anti-progestomimetischen Eigenschaften zumindest eine Verbindung der Formel $I'_B$

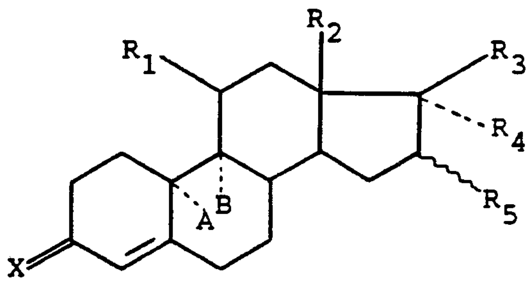

$I'_B$

umfaßt, worin entweder $R_1$ einen gegebenenfalls substituierten Thienylrest, einen Furylrest, einen Cycloalkylrest mit 3 bis 6 Kohlenstoffatomen, einen Phenylrest, gegebenenfalls substituiert durch einen oder mehrere Rest, ausgewählt unter den Hydroxy-, Halogen-, Trifluormethyl-, Alkyl, Alkoxy-, Alkoxyalkyl-, Alkylthio- oder Phenylthioresten, gegebenenfalls oxidiert in Form des Sulfoxids oder des Sulfons oder Alkenyloxy mit höchstens 6 Kohlenstoffatomen und Phenyloxy, bedeutet oder $R_1$ einen Naphthyl- oder Diphenylrest oder einen Alkyl- oder Alkenylrest, der gegebenenfalls mehrere Unsättigungen enthält, mit höchstens 6 Kohlenstoffatomen bedeutet,

$R_2$ für einen Methyl- oder Ethylrest steht,

$R_3$ für ein Wasserstoffatomen, eine gegebenenfalls substituierte Alkyl- oder Alkenylgruppe, eine Hydroxyl-, Acetyl-, Hydroacetyl-Gruppe, eine Carboxylalkoxygruppe mit 2 bis 4 Kohlenstoffatomen, die gegebenenfalls verestert oder in ein Salz überführt worden ist, oder eine gegebenenfalls veresterte Hydroxyalkylgruppe steht,

$R_4$ entwender ein Wasserstoffatom, eine Hydroxylgruppe, eine Alkyl-, Alkenyl- oder Alkinylgruppe mit höchstens 12 Kohlenstoffatomen, die gegebenenfalls substituiert ist durch eine Alkylamino-, Dialkylaminogruppe oder durch ein Halogenatom, eine Alkylthio-, Alkoxy- oder Trialkylsilylgruppe wiedergibt oder $R_4$ für eine Cyanomethylgruppe steht,

$R_5$ ein Wasserstoffatom oder einem Methylrest in α- oder β-Stellung bedeutet,

X für ein Sauerstoffatom oder eine Hydroxyimino- oder Alkoxyiminogruppe mit 1 bis 4 Kohlenstoffatomen in syn- oder anti-Stellung steht,

A und B eine α-Epoxyfunktion oder die Anwesenheit einer zweiten Bindung zwischen den Kohlenstoffatomen in 9- und 10-Stellung anzeigen, sowie die Salze dieser Produkte mit Säuren, wenn $R_4$ für einen eine Aminofunktion enthaltenden Rest steht.

8. Produkt gemäß den Ansprüchen 6 und 7, dadurch gekennzeichnet, daß der Substituent $R_1$ einen Phenylrest, substituiert in para-Stellung durch ein Halogenatom, einen Alkylrest mit 1 bis 4 Kohlenstoffatomen, einen Alkyloxy-, Alkylthio- oder Alkenyloxyrest mit höchstens 4 Kohlenstoffatomen, einen Phenyl- oder Phenylthiorest oder einen gegebenenfalls acylierten Hydroxylrest bedeutet, $R_3$ für einen Hydroxylrest steht und $R_4$ einen Alkinylrest mit höchstens 4 Kohlenstoffatomen wiedergibt, oder $R_3$ und $R_4$

einen Rest 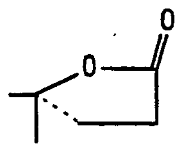 oder 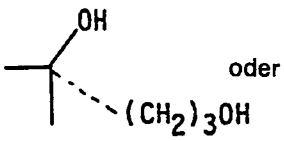 oder 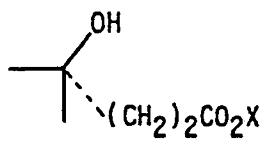

bilden, wobei X die in Anspruch 6 angegebene Bedeutung bestizt.

9. Produkt gemäß Anspruch 1, dadurch gekennzeichnet, daß es als Substanz mit anti-progestomimetischen Eigenschaften zumindest ein Steroid, ausgewählt unter den folgenden Verbindungen; enthält:
11β-[4-(N,N-Dimethylaminoethyloxy)phenyl]-17β-hydroxy-17α-(prop-1-inyl)-östra-4,9-dien-3-on;
11β-(4-Dimethylaminophenyl)-17β-hydroxy-17α-(prop-1-inyl)-östra-4,9-dien-3-on;
N-Oxid von 21-Chlor-17β-hydroxy-11β-(4-dimethylaminophenyl)-(17α)-19-nor-pregna-4,9-dien-20-in-on;
N-Oxid von 21-Chlor-9α,10α-epoxy-17β-hydroxy-11β-(4-dimethylaminophenyl)-(17α)-19-nor-pregn-4-en-20-in-3-on;
17β-Hydroxy-11β-(4-dimethylaminophenyl)-17α-(prop-2-inyl)-östra-4,9-dien-3-on;
N-Oxid von 17β-Hydroxy-11β-(4-dimethylaminophenyl)-17α-(prop-1-inyl)-östra-4,9-dien-3-on;
11β-[(4-Chlor)-phenyl]-17β-hydroxy-17α-(prop-1-inyl)-östra-4,9-dien-3-on;
11β-[(5-Chlor)-thienyl]-17β-hydroxy-17α-(prop-1-inyl)-östra-4,9-dien-3-on;
11β-[(3-Chlor)-phenyl]-17β-hydroxy-17α-(prop-1-inyl)-östra-4,9-dien-3-on;
11β-[(4-Methylthio)-phenyl]-17β-hydroxy-17α-(prop-1-inyl)-östra-4,9-dien-3-on;
11β-[(3-Fluor)-phenyl]-17β-hydroxy-17α-(prop-1-inyl)-östra-4,9-dien-3-on;
11β-[(4-Methylthio)-phenyl]-17α-methyl-19-nor-pregna-4,9-dien-3,20-dion;
11β-[(4-Methylthio)-phenyl]-16α-methyl-19-nor-pregna-4,9-dien-3,20-dion;
11β-Cyclopropyl-17β-hydroxy-17α-(prop-1-inyl)-östra-4,9-dien-3-on;
11β-3-(2-Propenyloxy)-phenyl)-17β-hydroxy-17α-(prop-1-inyl)-östra-4,9-dien-3-on;
9α,10α-Epoxy-17β-hydroxy-11β-(4-methoxyphenyl)-17α-(prop-1-inyl)-östr-4-en-3-on;
9α,10α-Epoxy-17β-hydroxy-11β-[(4-methylsulfonyl)-phenyl)-17α-(prop-1-inyl)-östra-4-en-3-on;
11β-[(3-Fluor)-phenyl]-3-hydroxyimino-17α-(prop-1-inyl)-östra-4,9-dien-17β-ol-anti-Isomeres;
17β-Hydroxy-17α-(prop-1-inyl)-11β(4-hydroxyphenyl)-östra-4,8-dien-3-on;
3-[11β-(4-Dimethylaminophenyl)-17β-hydroxy-3-oxo-4,9(10)-östradien-17α-yl]-propionsäure-lacton und das Kaliumsalz der entsprechenden Propionsäure;
11β-(4-Dimethylaminophenyl)-17β-hydroxy-17α-(3-hydroxypropyl)-4,9(10)-östradien-3-on;
Lacton der 3-[11β-(4-(2-Dimethylaminoethoxy)-phenyl]-17β-hydroxy-18-methyl-3-oxo-4,9(10)-östradien-17α-yl]-propionsäure und das Kalumsalz der entsprechenden Propionsäure;
11β-[4-(2-Dimethylaminethoxy)-phenyl]-17β-hydroxy-17α-(3-hydroxypropyl)-18-methyl-4,9(10)-östradien-3-on;

17α-Ethinyl-11β-(4-alloxyphenyl)-17β-hydroxy-4,9-östradien-3-on;
11β-(4-Alloxyphenyl)-17β-hydroxy-17α-(propinyl)-4,9-östradien-3-on.

10. Produkt gemäß den Ansprüchen 1 und 9, dadurch gekennzeichnet, daß es als Substanz mit anti-progestomimetischen Eigenschaften die Verbindung der Formel 11β-(4-Dimethylaminophenyl)-17β-hydroxy-17α-(prop-1-inyl)-östra-4,9-dien-3-on enthält.

11. Produkt gemäß Anspruch 1, dadurch gekennzeichnet, daß es als Substanz mit uterotonischen Eigenschaften zumindest eine der Substanzen unter Oxytocin, den Ergotalkaloiden des Mutterkorns und Spartein enthält.

## Claims

1. Product containing at least one substance possessing anti-progesterone or anti-progestomimetic properties and at least one substance different to a prostaglandine and possessing uterotonic properties as a combination product, which can be used simultaneously, separately or spread out over time for the interruption of pregnancy.

Product according to claim 1 characterized in that it contains as a substance possessing anti-progestomimetic properties, at least one compound corresponding to the formula $I_A$:

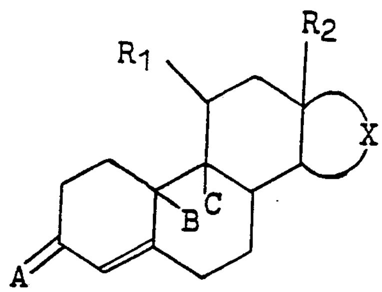

$(I_A)$

in which $R_1$ represents an organic radical containing 1 to 18 carbon atoms, containing at least one nitrogen, phosphorus or silicon atom, the atom immediately adjacent to the carbon in position 11 being a carbon atom, $R_2$ represents a hydrocarbonated radical containing 1 to 8 carbon atoms, X represents the remainder of a pentagonal or hexagonal cycle optionally substituted and optionally carrying an unsaturation, the C=A group in position 3 represents an oxo group, free or blocked in the form of a ketal group, a

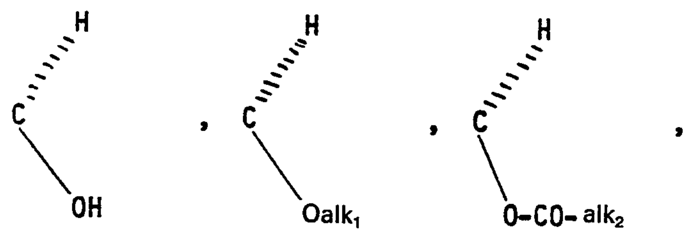

group, a C=NOH group, a C=NO-alk$_3$ group, or a $CH_2$ group, alk$_1$, alk$_2$ and alk$_3$ represent an alkyl radical containing 1 to 8 carbon atoms or an aralkyl group containing 7 to 15 carbon atoms and B and C together form a double bond or an epoxide bridge, as well as their addition salts with acids.

3. Product according to claims 1 or 2 characterized in that in formula $I_A$, the remainder X is chosen from the cycles of formula:

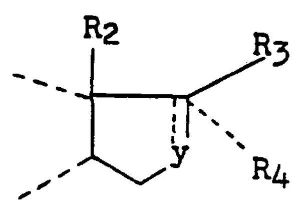

in which $R_2$ retains the same meaning as in claim 2, the dotted line in position 16—17 indicates the possible presence of a double bond, Y represents a

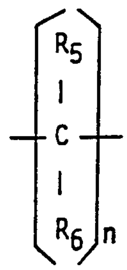

radical, in which n represents the number 1 or 2, $R_5$ represents a hydrogen atom, an alkyl radical containing 1 to 8 carbon atoms, an alkenyl radical or an alkynyl radical containing 2 to 8 carbon atoms, an aryl radical containing 6 to 14 carbon atoms or an aralkyl radical containing 7 to 15 carbon atoms, $R_6$, identical or different to $R_5$, can take one of the values indicated for $R_5$ and can also represent a hydroxyl radical, $R_3$ and $R_4$, identical or different, represent either a hydrogen atom, or an OH, $Oalk_4$, O—CO-$alk_5$ radical, $alk_4$ and $alk_5$ representing any alkyl radical containing 1 to 8 carbon atoms or aralkyl radical containing 7 to 15 carbon atoms, or an alkenyl radical or an alkynyl radical containing 2 to 8 carbon atoms, or a

$$-\overset{\overset{\displaystyle O}{\|}}{C}-CH_2OH$$

radical, or a $-COCH_2OCOalk_6$ radical, in which $alk_6$ represents an alkyl radical containing 1 to 8 carbon atoms optionally substituted or an aralkyl radical containing 7 to 15 carbon atoms, or a $CO-CO_2H$ radical, or a $CO-CO_2-alk_7$ radical in which $alk_7$ represents an alkyl radical containing 1 to 8 carbon atoms, or a

$$-\overset{\overset{\displaystyle H}{|}}{C}=O$$

radical or a

$$-\overset{\overset{\displaystyle NHalk_8}{|}}{C}=O$$

radical, in which $alk_8$ represents an alkyl radical containing 1 to 8 carbon atoms or an aralkyl radical containing 7 to 15 carbon atoms, or a $-C\equiv N$ radical, or $R_3$ and $R_4$ together form

a radical 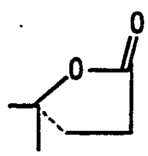 , a 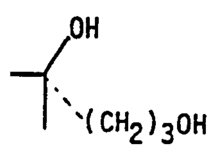 radical

or a 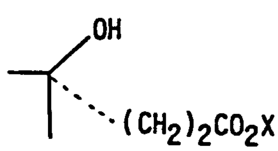 radical

in which X represents a hydrogen, sodium or potassium atom.

4. Product according to claims 1 to 3 characterized in that, in formula $I_A$, the remainder X is chosen from the cycles of formula:

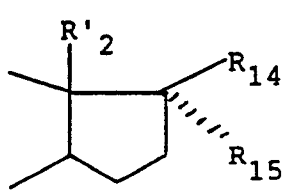

in which $R'_2$ represents a methyl or ethyl radical, $R_{14}$ represents a hydroxyl radical, $R_{15}$ represents an alkynyl radical having 2 to 4 carbon atoms or $R_{14}$ and $R_{15}$ together form a:

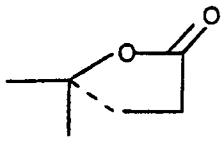 ou 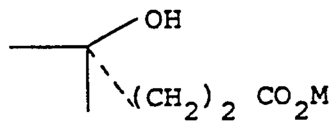

group, in which M represents a potassium or sodium atom.

5. Product according to claims 1 to 4 characterized in that, in formula $I_A$, $R_1$ represents a 2, 3 or 4-pyridyl, a

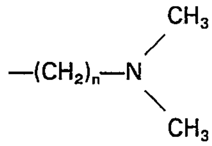 radical ($n \geq 3$),

a 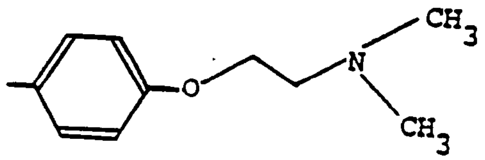 radical,

a 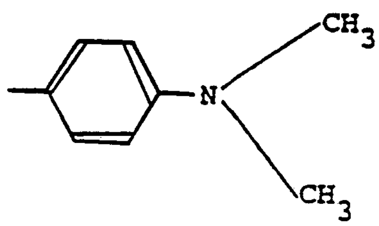 radical

a 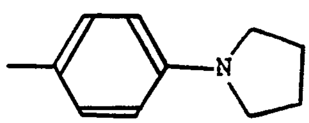 radical

a 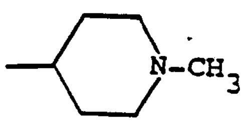 radical;

the nitrogen atoms being optionally oxidized.

6. Product according to claim 1, characterized in that it contains as substance possessing anti-progestomimetic properties, at least one compound corresponding to formula $I_B$:

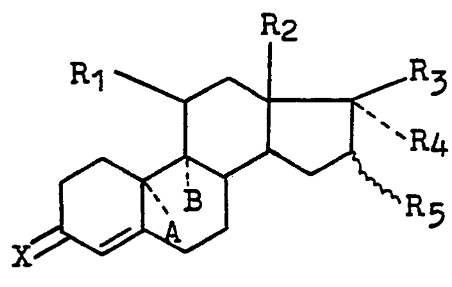 $I_B$

in which either $R_1$ represents an optionally substituted thienyl radical, a furyl radical, a cycloalkyl radical having 3 to 6 carbon atoms; a phenyl radical optionally substituted by one or more radicals chosen from the following radicals: hydroxy, halogen, trifluoromethyl, alkyl, alkoxy, alkoxyalkyl, alkylthio or phenylthio, optionally oxidized in the form of the sulphoxide or sulphone or alkenyloxy having at most 6 carbon atoms and phenyloxy;

or $R_1$ represents a naphtyl or diphenyl radical or an alkyl radical or an alkenyl radical optionally carrying several unsaturations having at most 6 carbon atoms;

$R_2$ represents a methyl or ethyl radical,

$R_3$ represents a hydrogen atom, an alkyl radical, an alkenyl radical optionally substituted, a hydroxyl radical, acetyl radical, hydroxyacetyl, carboxyalkoxy radical having 2 to 4 carbon atoms optionally esterified or salified, hydroxyalkyl radical optionally esterified,

$R_4$ represents either a hydrogen atom, a hydroxyl radical, an alkyl radical, an alkenyl radical or an alkynyl radical having at most 12 carbon atoms optionally substituted by an alkylamino radical, a dialkyl amino radical or by a halogen, an alkylthio radical, an alkoxy radical or a trialkylsilyl radical, or $R_4$ represents a cyanomethyl radical, or $R_3$ and $R_4$ together represent a

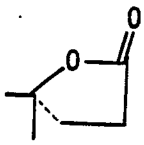 radical a 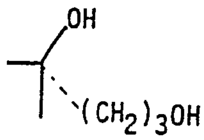 radical

or a 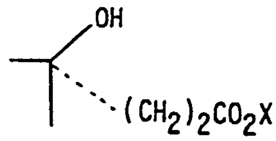 radical

in which X represents a hydrogen, sodium or potassium atom.

$R_5$ represents a hydrogen atom or a methyl radical in position alpha or beta,

X represents an oxygen atom or a hydroxyimino radical or an alkoxyimino radical having 1 to 4 carbon atoms in syn or anti position;

A and B represent an alpha-epoxy function or the presence of a second bond between carbons 9 and 10, as well as the salts of these products with acids when $R_4$ represents a radical including an amino function.

7. Product according to claims 1 to 6 characterized in that they contain, as substance possessing anti-progestomimetic properties, at least one compound corresponding to the formula $I'_B$:

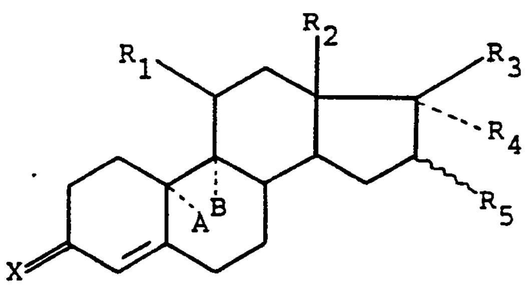

$I'_B$

in which either $R_1$ represents an optionally substituted thienyl radical, a furyl radical; a cycloalkyl radical having 3 to 6 carbon atoms; a phenyl radical optionally substituted by one or more radicals chosen from the radicals hydroxy, halogen, trifluoromethyl, alkyl, alkoxy, alkoxyalkyl, alkylthio or phenylthio optionally oxidized in the form of the sulphoxide or the sulphone or alkenyloxy having at most 6 carbon atoms, and phenyloxy radical,

or $R_1$ represents a naphthyl or diphenyl radical or an alkyl radical or an alkenyl radical optionally carrying several unsaturations having at most 6 carbon atoms;

$R_2$ represents a methyl or ethyl radical,

$R_3$ represents a hydrogen atom, an alkyl radical, an optionally substituted alkenyl radical, a hydroxyl radical, an acetyl radical, a hydroxyacetyl radical, a carboxyalkoxy radical having 2 to 4 carbon atoms optionally esterified or salified, a hydroxyalkyl radical optionally esterified,

$R_4$ represents either a hydrogen atom, a hydroxyl radical, an alkyl radical, an alkenyl radical, or an alkynyl radical having at most 12 carbon atoms optionally substituted by an alkylamino radical, a dialkyl amino radical or by a halogen, an alkylthio radical, an alkoxy radical or a trialkylsilyl radical, or $R_4$ represents a cyanomethyl radical.

$R_5$ represents a hydrogen atom or a methyl radical in alpha or beta position,

X represents an oxygen atom or a hydroxyimino radical or an alkoxyimino radical having 1 to 4 carbon atoms in syn or anti position;

A and B represent an alpha epoxy function or the presence of a second bond between the carbons in positions 9 and 10, as well as the salts of these products with acids when $R_4$ represents a radical including an amino function.

8. Product according to claims 6 and 7 characterized in that the substituent $R_1$ represents a phenyl radical substituted in para position by a halogen atom, an alkyl radical having from 1 to carbon atoms, an alkoxy radical, an alkylthio radical, or an alkenyloxy radical having at most 4 carbon atoms, a phenyl radical or phenylthio radical or a hydroxyl radical optionally acylated,

$R_3$ represents a hydroxyl radical and $R_4$ represents an alkynyl radical having at most 4 carbon atoms, either $R_3$ and $R_4$

represent a 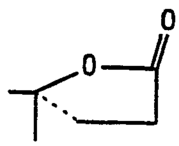 radical or 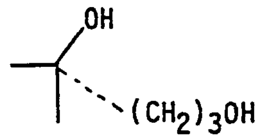 or 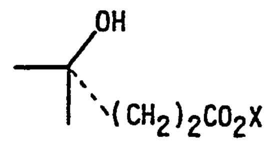

X having the value indicated in claim 6.

9. Product according to claim 1 characterized in that it contains as substance possessing anti-progestomimetic properties, at least one steroid chosen from the following compounds:
11beta-[4-(N,N-dimethyl-amino-ethyloxy)-phenyl]-17beta-hydroxy-17alpha-(prop-1-ynyl)-estra-4,9-dien-3-one;
11beta-(4-dimethyl-amino-phenyl)-17beta-hydroxy-17alpha-(prop-1-ynyl)-estra-4,9-dien-3-one;
N-oxide of 21-chloro-17beta-hydroxy-11beta-(4-dimethylaminophenyl)-(17alpha)-19-nor-pregna-4,9-dien-20-yn-3-one;
N-oxide of 21-chloro-9alpha,10alpha-epoxy-17beta-hydroxy-11beta-(4-dimethylaminophenyl)-(17alpha)-19-nor-pregna-4-en-20-yn-3-one;
17beta-hydroxy-11beta-(4-dimethylaminophenyl)-17alpha-(prop-2-ynyl)-estra-4,9-dien-3-one;
N-oxide of 17beta-hydroxy-11beta-(4-dimeethylaminophenyl)-17alpha-(prop-1-ynyl)-estra-4,9-dien-one;
11beta-[(4-chloro)-phenyl]-17beta-hydroxy-17alpha-(prop-1-ynyl)-estra-4,9-dien-3-one;
11beta-[(5-chloro)-thienyl]-17beta-hydroxy-17alpha-(prop-1-ynyl)-estra-4,9-dien-3-one;
11beta-[(3-chloro)-phenyl]-17beta-hydroxy-17alpha-(prop-1-ynyl)-estra-4,9-dien-3-one;
11beta-[(4-methylthio)-phenyl]-17beta-hydroxy-17alpha-(prop-1-ynyl)-estra-4,9-dien-3-one;
11beta-[(3-fluoro)-phenyl]-17beta-hydroxy-17alpha-(prop-1-ynyl)-estra-4,9-dien-3-one;
11beta-[(4-methylthio)-phenyl]-17alpha-methyl-19-nor,pregna-4,9-dien-3,20-di-one;
11beta-[(4-methylthio)-phenyl]-16alpha-methyl-19-nor,pregna-4,9-dien-3,20-di-one;
11beta-cyclopropyl-17beta-hydroxy-17alpha-(prop-1-ynyl)-estra-4,9-dien-3-one;
11beta-3(2-propenyloxy)-phenyl-17beta-hydroxy-17alpha-(prop-1-ynyl)-estra-4,9-dien-3-one;
9alpha, 10alpha-epoxy-17beta-hydroxy-11beta-(4-methoxyphenyl)-17alpha-(prop-1-ynyl)-estr-4-dien-3-one;
9alpha, 10alpha-epoxy-17beta-hydroxy-11beta[(4-methylsulphonyl)-phenyl]-17alpha-(prop-1-ynyl)-estra-4-dien-3-one;
11beta-[(3-fluoro)-phenyl]-3-hydroxyimino-17alpha-(prop-1-ynyl)-estra-4,9-dien-17beta-ol anti isomer;
17beta-hydroxy-17alpha-(prop-1-ynyl)-11beta-(4-hydroxyphenyl)-estra-4,9-dien-3-one;
Lactone of 3-[11beta-(4-dimethylaminophenyl)-17beta-hydroxy-3-oxo-4,9(10)-estradien-17alpha-yl] propionic acid and the corresponding potassium salt of propionic acid;
11beta-(4-dimethylaminophenyl)-17beta-hydroxy-17alpha-(3-hydroxypropyl)-4,9(10)-estradien-3-one;
Lactone of 3-[11beta[4-(2-dimethylaminoethoxy)-phenyl]-17beta-hydroxy-18-methyl-3-oxo-4,9(10)-estradien-17alpha-yl]propionic acid and the corresponding potassium salt of propionic acid;
11beta-[4-(2-dimethylaminoethoxy)-phenyl]-17beta-hydroxy-17-alpha-(3-hydroxypropyl)-18-methyl-4,9(10)-estradien-3-one;
17alpha-ethynyl-11beta-(4-alloxyphenyl)-17beta-hydroxy-4,9-estradien-3-one;
11beta-(4-alloxyphenyl)-17beta-hydroxy-17alpha-(1-propynyl)-4,9-estradien-3-one.

10. Product according to claims 1 to 9 characterized in that it contains, as substance possessing anti-progestomimetic properties, the compound corresponding to the formula: 11beta-(4-dimethylaminophenyl)-17beta-hydroxy-17alpha-(prop-1-ynyl)-estra-4,9-dien-3-one.

11. Product according to claim 1 characterized in that it contains as substance possessing uterotonic properties, at least one of the substances comprised by ocytocin, the rye ergot alkaloids and sparteine.